Europäisches Patentamt

European Patent Office

Office européen des brevets

(11) Numéro de publication : **0 110 787**
**B1**

# (12) FASCICULE DE BREVET EUROPÉEN

(45) Date de publication du fascicule du brevet :
**15.03.89**

(21) Numéro de dépôt : **83402283.2**

(22) Date de dépôt : **25.11.83**

(51) Int. Cl.⁴ : **G 06 F 15/62**

(54) **Procédé et appareil pour former des images en coupe d'un corps.**

(30) Priorité : **26.11.82 US 444613**

(43) Date de publication de la demande :
**13.06.84 Bulletin 84/24**

(45) Mention de la délivrance du brevet :
**15.03.89 Bulletin 89/11**

(84) Etats contractants désignés :
**DE FR GB IT NL**

(56) Documents cités :
**MEDICAL PHYSICS, vol. 5, no. 6, novembre/décembre 1978, pages 485-490, Am. Assoc. Phys. Med., New York, US; J.E. HOLDEN et al.: "Continuous time-dependence in computed tomography"**

(73) Titulaire : **GENERAL ELECTRIC CGR SA.**
**13, Square Max-Hymans**
**F-75015 Paris (FR)**

(72) Inventeur : **Kruger, Robert A.**
**THOMSON-CSF SCPI 173, bld Haussmann**
**F-75379 Paris Cedex 08 (FR)**
Inventeur : **Nelson, James A.**
**THOMSON-CSF SCPI 173, bld Haussmann**
**F-75379 Paris Cedex 08 (FR)**

(74) Mandataire : **Ballot, Paul Denis Jacques et al**
**Cabinet Ballot-Schmit 84, avenue Kléber**
**F-75116 Paris (FR)**

Jouve, 18, rue St-Denis, 75001 Paris, France

## Description

La présente invention se rapporte à un procédé de formation d'images de la structure interne d'un corps et elle a trait plus particulièrement à un procédé et à un appareil pour obtenir des images dans des coupes d'un corps.

En tomographie conventionnelle, l'objectif est classiquement d'obtenir une image d'une ou plusieurs coupes ou plans de la structure interne d'un corps par combinaison d'une information à partir d'un certain nombre d'images obtenues dans différentes perspectives angulaires en vue d'obtenir une seule image traitée pour chaque coupe. Typiquement, les images multiples sont traitées et additionnées par un ordinateur ou photographiquement pour obtenir finalement une seule image traitée. Le processus peut ensuite être répété pour d'autres coupes ou plans à l'intérieur du corps.

Il est bien connu qu'une série d'images radiologiques peuvent être observées pour visualiser la progression d'une dose d'un produit de contraste, appelée « bolus », dans un vaisseau sanguin examiné. On utilise des agents de contraste radiographique pour créer une grande différence de comportement d'absorption de rayons X. Des vaisseaux sanguins sont virtuellement invisibles sur les images radiologiques (excepté dans la poitrine) du fait que du sang, un muscle, de la graisse et un tissu mou possèdent tous un comportement semblable d'absorption de rayons X. Des agents de contraste radiographique contiennent un produit possédant des propriétés d'absorption de rayons X qui sont différentes de celles du sang, d'un muscle, de graisse et d'un tissu mou. Par exemple, lorsqu'un « bolus » d'un produit de contraste formé par un liquide iodé est injecté dans une artère ou une veine, la structure vasculaire produit artificiellement un plus fort contraste sur une image radiographique pendant que le produit de contraste se trouve à l'intérieur d'un certain segment vasculaire.

Récemment, on a mis au point différentes techniques pour traiter des images radiologiques, par exemple avec des filtres temporels pour obtenir des images améliorées. Cependant, même ces images améliorées peuvent avoir une utilité limitée dans certaines applications, par exemple lorsque des éléments vasculaires placés les uns sur les autres ou d'autres parties anatomiques limitent l'observation. Dans de tels cas, il est souvent nécessaire ou souhaitable d'obtenir une image en coupe.

Il rentre parmi les objets de la présente invention de produire des images de coupes d'un corps par un type de tomographie qui représente, en temps réel, une séquence de trames vidéo représentant un plan du corps à examiner. Il rentre également parmi les objets de la présente invention de créer un appareil et un procédé permettant de représenter des séquences d'images traitées correspondant à différents plans intéressants du corps, sans qu'il soit nécessaire de soumettre un patient à une nouvelle séquence d'expositions pour chaque plan à représenter. Il rentre également parmi les objets de la présente invention d'utiliser des procédés de formation d'images radiologiques et de traitement dans un système tomographique pour obtenir des avantages par rapport à des techniques tomographiques et radiographiques existantes.

La présente invention concerne un procédé et un appareil pour produire une image traitée d'une coupe d'un corps. Dans la présente invention, on réalise une tomographie dans laquelle une série de trames vidéo d'une coupe d'un corps peuvent être observées en temps réel. De cette manière par exemple, on peut contrôler la progression d'un « bolus » d'un produit de contraste se déplaçant dans un vaisseau sanguin dans la coupe en train d'être examinée. Dans une forme de l'invention, une série enregistrée de trames d'un plan principal d'un corps en train d'être examiné peut être traitée pour obtenir une série correspondante de trames qui représentent un plan intéressant qui est espacé du plan principal, en évitant ainsi l'obligation d'effectuer une ré-exposition pour chaque nouveau plan à observer.

Conformément au procédé de l'invention, un corps est disposé entre un ensemble formé par une source de rayonnement et un détecteur associé, de manière qu'un faisceau de rayonnement émis par la source arrive angulairement sur le corps et passe au travers en direction du détecteur. Un mouvement relatif de rotation est établi entre le faisceau et le corps (par exemple en faisant déplacer l'ensemble source-détecteur, le corps ou bien les deux) de manière qu'un plan principal intéressant du corps reste essentiellement au foyer pendant le mouvement relatif de rotation. Une série de trames de signaux vidéo électroniques est obtenue à partir du détecteur, pour différentes positions angulaires, les trames représentant les images des caractéristiques de transmission de rayonnement du corps à une série d'instants successifs. Les trames de la série sont filtrées temporellement et ensuite affichées.

Dans le mode préféré de réalisation de l'invention, l'étape de filtrage temporel consiste à filtrer la série de trames de signaux vidéo à partir d'une fonction de filtrage ayant une réponse de fréquence temporelle qui correspond sensiblement à la fréquence temporelle du mouvement d'un « bolus » d'une matière de contraste dans la région en train d'être observée. De préférence, cette réponse temporelle devient très petite à un instant qui est inférieur au temps de rotation caractéristique du système de tomographie. De cette manière, le filtre temporel agit de façon à éliminer par filtrage le mouvement tomographique, ainsi que certains autres types de mouvement et, si on le désire une anatomie stationnaire.

Dans une forme de l'invention, chaque trame de la série de trames obtenues comprend un groupe d'éléments élémentaires d'images appelés pixels, le niveau de signal vidéo pour chaque pixel étant déterminé par la transmissivité de rayonnement d'une région élémentaire du plan principal et de plans adjacents au travers desquels passent les rayons du faisceau. Pour former une image d'un plan intéressant autre que le plan focal principal, des transformations géométriques sont réalisées pour décaler les niveaux du signal vidéo dans différentes positions de pixels, les transformations étant fonction de l'angle relatif de rotation associé à la trame contenant le pixel, et de la distance entre le plan intéressant à représenter et le plan principal.

D'autres caractéristiques et avantages de l'invention seront mis en évidence dans la suite de la description donnée à titre d'exemple non limitatif, en référence aux dessins annexés dans lesquels :

- la figure 1 est un schéma-bloc d'un appareil conforme à l'invention qui peut être utilisé pour la mise en oeuvre du procédé de l'invention ;

- la figure 2 est un diagramme simplifié de rayons du faisceau de rayonnement, qui est utile pour la compréhension du fonctionnement d'une partie caractéristique d'un mode de réalisation de l'invention ;

- la figure 3 est un graphique montrant la variation temporelle associée à un point focal ;

- la figure 4 est un graphique qui montre la variation temporelle associée à un point focal ;

- la figure 5 est un graphique qui donne les fréquences temporelles associées à un point focal ;

- la figure 6 est un graphique donnant les fréquences temporelles associées à un point non focal ;

- la figure 7 est un graphique donnant la variation temporelle associée à un vaisseau dans un plan focal ;

- la figure 8 est un graphique donnant la variation temporelle associée à un vaisseau dans un plan non focal ;

- la figure 9 est un graphique donnant les fréquences temporelles associées à un vaisseau se trouvant dans un plan focal ;

- la figure 10 est un graphique donnant les fréquences temporelles associées à un vaisseau se trouvant dans un plan non focal ;

- la figure 11 est un organigramme d'un programme de commande du processeur du mode de réalisation de la Figure 1, servant à assurer un décalage d'adresses de pixels pour observer un plan intéressant autre que le plan principal ;

- la figure 12 est un schéma bloc d'un système de filtrage temporel qui peut être utilisé dans le mode de réalisation de la Figure 1.

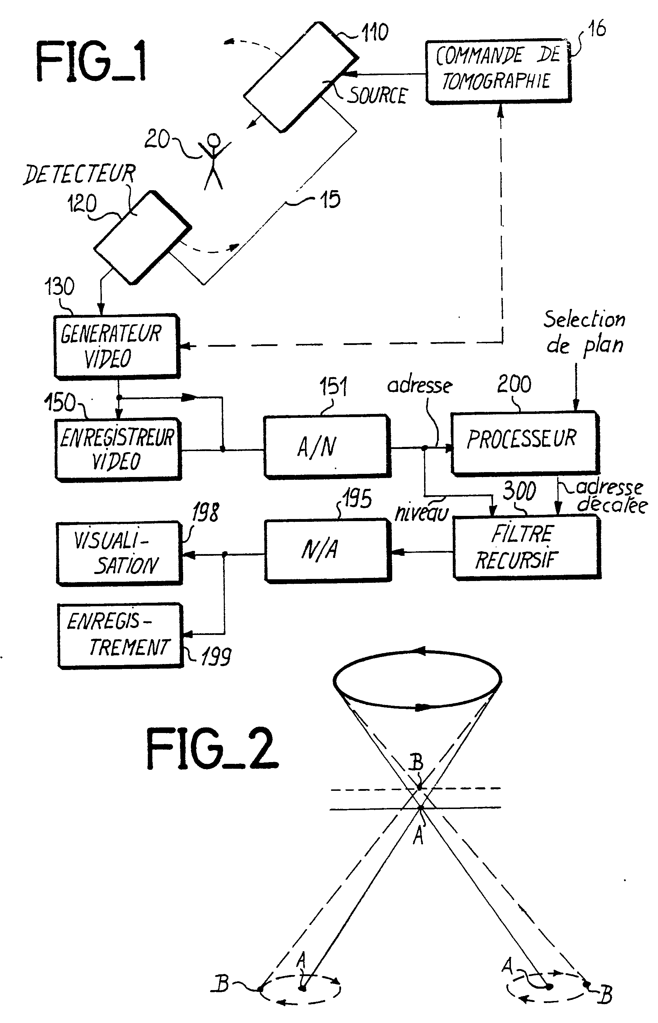

Sur la figure 1, on a représenté un schéma-bloc d'un mode de réalisation d'un appareil 100 pour obtenir des images en coupe de la structure interne d'un corps 20. L'appareil 100 comprend un mécanisme conventionnel de tomographie circulaire ou elliptique 15, modifié pour une application radioscopique. Une source de rayonnement 110, typiquement une source de rayons X, et un détecteur 120 sont montés de manière à tourner d'une façon coordonnée, par exemple autour d'un axe du corps 20, sous la commande d'un entraînement tomographique 18. Le mécanisme de tomographie peut être du type fabriqué par CGR Médical Corp. of Baltimore Maryland. Un générateur vidéo 130 fonctionne en combinaison avec le détecteur 120. La combinaison du détecteur et du générateur vidéo peut comprendre par exemple un élément d'intensification d'image de rayons X en coopération avec une caméra de télévision. La sortie du générateur vidéo 130 est reliée à un enregistreur vidéo 150 et également à un convertisseur analogique-numérique 151, qui convertit le signal de télévision sous une forme numérique et qui engendre des adresses séquentielles. La sortie de l'enregistreur 150 est également reliée au convertisseur 151. Un équipement permettant d'obtenir le signal de télévision converti numériquement est bien connu dans l'art antérieur et commercialement disponible, un exemple étant constitué par le modèle AD-964310 fabriqué par THOMSON-CSF Broadcast, Inc. Pour chaque pixel de la trame vidéo, le convertisseur numérique de signal de télévision engendre un signal numérique, par exemple un signal numérique de huit bits, représentant une des 256 gradations de niveau de luminance (pour un signal monochrome — comme considéré dans le mode de réalisation représenté), en même temps qu'une adresse qui définit la position du pixel. L'enregistreur vidéo 150 peut être tout dispositif d'enregistrement approprié, tel qu'un enregistreur vidéo sur bande ou sur disque. Le générateur vidéo, l'enregistreur vidéo et le convertisseur analogique-numérique reçoivent classiquement des signaux de synchronisation et le convertisseur analogique-numérique reçoit également des signaux d'horloge à la fréquence des pixels.

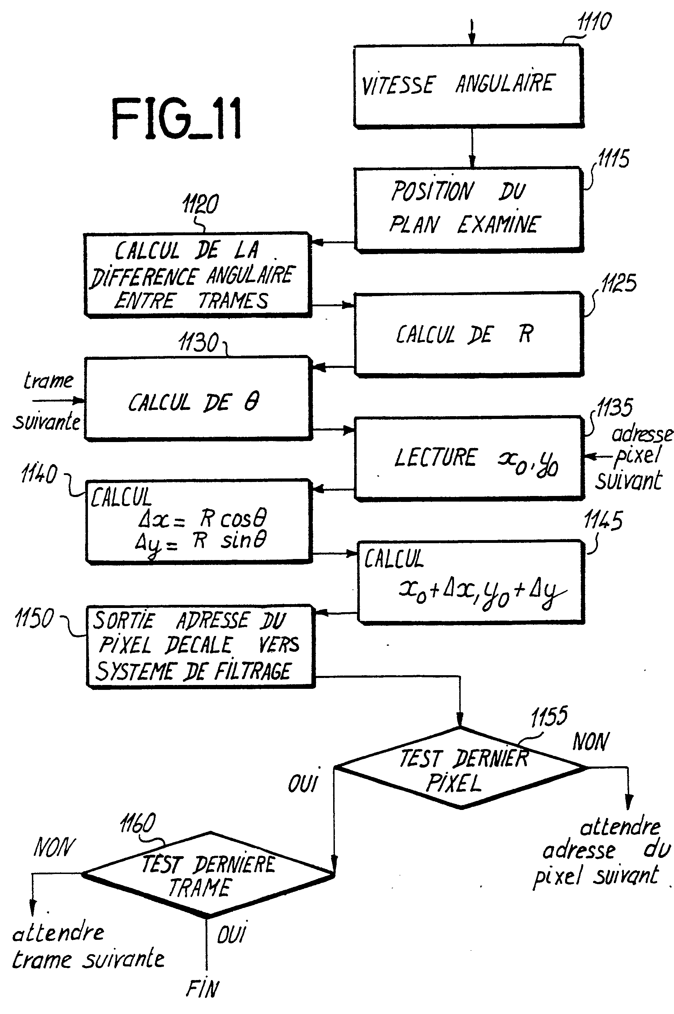

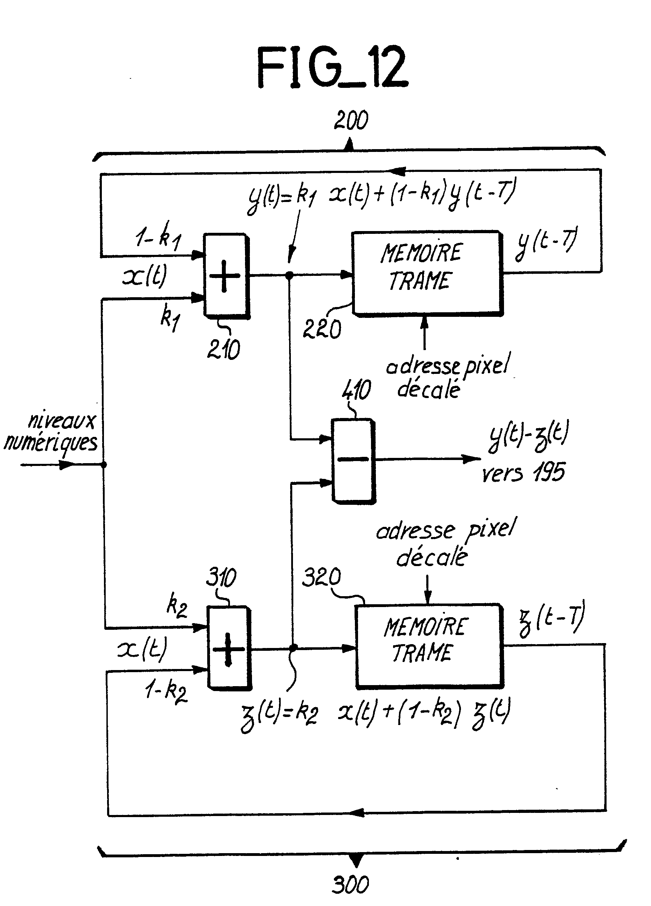

La sortie du convertisseur analogique-numérique 151 est appliquée à un circuit de décalage x, y 200, qui peut comprendre un processeur tel qu'un calculateur numérique ou un microprocesseur. Le processeur 200 peut comprendre un microprocesseur modèle 68000 fabriqué par Motorola Corp., ou bien un mode de réalisation de celui-ci appelé « tranche de bits ». Le processeur, qui peut être programmé en accord avec le programme décrit en référence à la figure 11, assure un décalage de l'adresse d'un pixel courant, l'adresse décalée du pixel étant appliquée au système de filtrage récurrent 300. Le niveau de signal numérique pour le pixel courant est également appliqué au sous-système de filtrage récurrent 300, qui est décrit en référence à la figure 12. Le signal de sortie du système de filtrage récurrent 300 est appliqué à un convertisseur numérique-analogique 195 dont le signal de sortie est appliqué à un dispositif d'affichage 198 et à un enregistreur vidéo 199. A nouveau, on a supposé que les signaux de synchronisation et des signaux d'horloge sont classiquement disponibles dans ces circuits.

Avant de poursuivre la description du fonctionnement du système, on va donner quelques considérations théoriques.

On va considérer un mouvement tomographique circulaire dans lequel, comme dans le cas considéré, le plan du film a été remplacé par une chaîne de télévision à intensification d'image. On va supposer que le mouvement circulaire se répète à une cadence d'une fois par seconde. L'angle tomographique n'est pas spécifié. La figure 2 montre la géométrie et la position de deux points dans un objet à trois dimensions à représenter en image, un point étant situé dans le plan principal (ou plan focal) et un second point étant situé dans un plan placé au-dessus. (Du fait que la surface d'entrée de l'élément d'intensification d'image n'est pas plane, des plans focaux sont en réalité des surfaces d'incurvation bidimensionnelle, d'une forme semblable à la surface d'entrée de l'élément d'intensification d'image. Puisque la courbure est faible, cette distorsion n'est pas considérée comme ayant une grande conséquence). On va supposer pour le moment que l'objet est stationnaire.

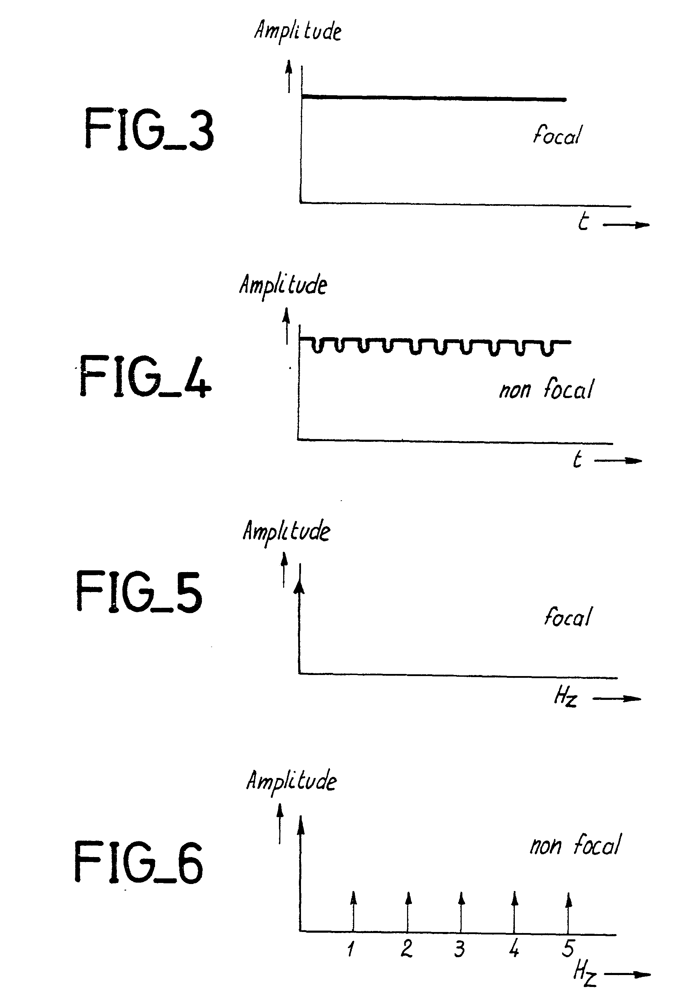

La projection du point focal tombe toujours sur le même pixel (le même point sur la surface de l'élément d'intensification d'image). La projection du point dans le plan situé au-dessus trace un cercle sur la surface d'élément d'intensification d'image. Plus l'éloignement d'un plan non focal est grand par rapport au plan focal, plus le diamètre de ce cercle est grand. Un pixel isolé placé le long de la trajectoire de ce point sur la surface d'élément d'intensification d'image « voit » ce point une fois par seconde. Les variations temporelles d'image associée à ces deux points sont indiquées respectivement sur les figures 3 et 4.

Maintenant, au lieu d'observer les variations temporelles des projections de ces points, on va considérer les composantes de fréquence temporelle qui sont associées à ces variations. Elles sont représentées sur les figures 5 et 6 et représentent respectivement les transformations de Fourier des figures 3 et 4. Le point focal fait intervenir seulement une composante de courant continu à une fréquence temporelle nulle. Le point non focal fait intervenir une composante de courant continu, et également des pics à ±1 cycle/s, et également les harmoniques supérieurs à ±n cycle/s, où n est un nombre entier.

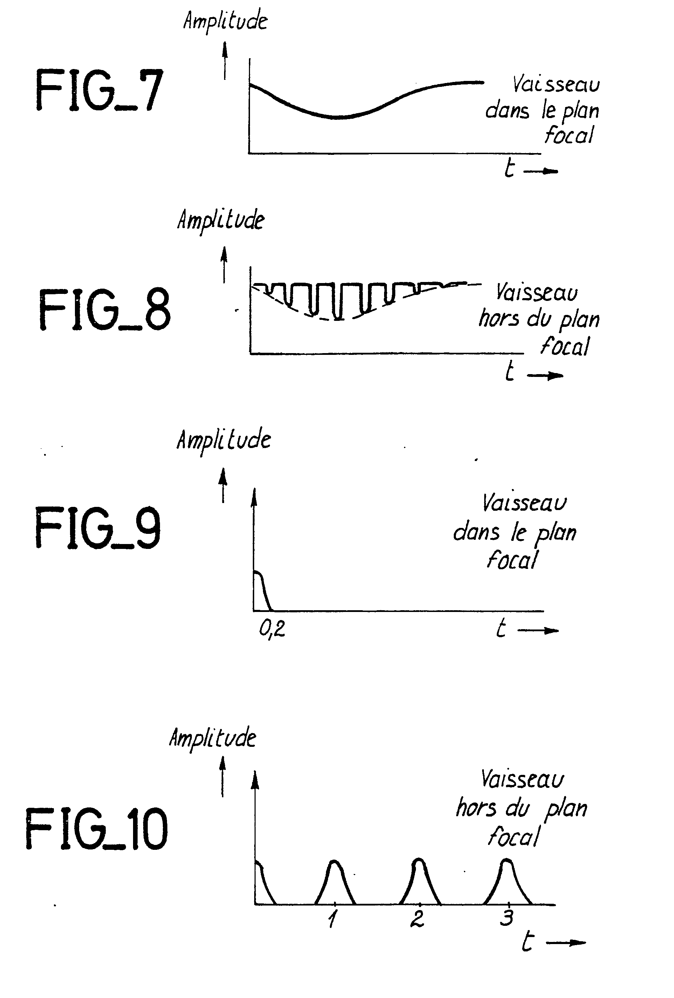

On va maintenant supposer que, à la place d'un objet stationnaire, il existe deux artères canalisant du sang opacifié par injection intraveineuse d'un produit de contraste. Un vaisseau passe par le point A et l'autre vaisseau par le point B. Les variations temporelles d'image représentées sur les figures 3 et 4 peuvent maintenant être considérées comme étant modifiées en celles représentées sur les figures 7 et 8. Les composantes de fréquences temporelles associées à ces variations sont représentées respectivement sur les figures 9 et 10.

La majeure partie des composantes de fréquences temporelles peuvent être éliminées de plans non focaux, tandis qu'on élimine simultanément une anatomie stationnaire de fond de tous les plans, en faisant passer les signaux vidéo provenant de la séquence tomographique au travers d'un système de filtrage récurrent, tel que le système de filtrage représenté sur la figure 12. La réponse de filtre pour une fréquence temporelle nulle est proche de zéro, et au-dessus de 1 Hz, elle est très petite. L'effet combiné d'un mouvement périodique rapide et d'un filtrage dans une bande passante consiste à éliminer la plupart des structures non artérielles et visualiser des artères se trouvant dans une zone du plan focal avec une bonne résolution, et également de former des images d'artères situées dans d'autres plans éloignés, sous la forme d'artères floues, le flou augmentant avec la distance d'éloignement par rapport au plan focal. D'autres filtres temporels peuvent être utilisés. Egalement le plan focal peut être modifié par déplacement du patient ou par mouvement pas à pas du mécanisme de tomographie.

Une limitation au processus de formation d'images décrit consiste en ce que, bien qu'il puisse être mis en œuvre en temps réel, on n'obtient une image que d'un seul plan. Cependant, comme indiqué ci-dessus, si la séquence d'images d'origine est mémorisée, on peut utiliser cette séquence de manière à synthétiser d'autres plans sans une nouvelle exposition du patient. Pendant le processus de saisie de données d'origine, des projections de point situés dans des plans non focaux tracent des trajectoires circulaires dans le plan du détecteur. En enregistrement, les positions angulaires associées à chaque trame vidéo convertie numériquement, on peut déterminer les décalages d'images (angles et rayons) associés à un plan donné pour une position angulaire spécifiée. Pour reconstituer les séquences dynamiques montrant un écoulement dans d'autres plans que le plan focal d'origine, on peut compenser de tels décalages sur une base trame-par-trame, en effectuant en fait le suivi d'une trajectoire caractéristique présélectionnée d'un plan particulier. Cela est réalisé dans le présent mode de réalisation par un déplacement de l'adressage numérique (x, y) en compensation des décalages prédéterminés.

Sur la figure 3, si le rayon du cercle associé au point B est R (qui est une fonction géométriquement connue de la distance entre les points A et B et de l'angle tomographique, $\phi$), et si l'angle de rotation, par rapport à une référence, est $\theta$, les déplacements x et y du point, désignés respectivement par x et y, peuvent alors être représentés par :

$$\Delta x = R\cos \theta \qquad (1)$$
$$\Delta y = R\sin \theta \qquad (2)$$

si les coordonnées initiales non décalées du point sont désignées par $x_o$, $y_o$, les coordonnées décalées x', y' s'écrivent alors :

$$x' = x_o — \Delta x \qquad (3)$$
$$y' = y_o — \Delta y$$

La figure 11 est un organigramme du programme de commande du processeur 200 (figure 1) pour assurer le décalage des adresses de pixels en vue de l'observation d'un plan intéressant autre que le plan principal. La vitesse angulaire du système tomographique est introduite (bloc 1110) et la position du plan intéressant, c'est-à-dire la distance et sa direction à partir du plan principal, est également introduite (bloc 1115). La différence angulaire entre les trames est ensuite obtenue par programme à partir de la vitesse angulaire introduite et du nombre de trames par seconde à utiliser, cette fonction étant représentée par le bloc 1120. Le rayon de base, R, est alors déterminé en fonction du plan considéré et de l'angle tomographique (bloc 1125). L'angle caractéristique $\theta$, est alors calculé pour la trame arrivant ensuite, par addition de la différence angulaire entre trames à l'accumulation d'angles (bloc 1130). Lorsque l'adresse suivante de pixel de la trame arrive, l'adresse prédécalée de pixel $x_o$, $y_o$ est lue (bloc 1135). Les relations (1) et (2) sont alors utilisées pour calculer les composantes de décalage $\Delta x$ et $\Delta y$, comme représenté par le bloc 1140. L'adresse décalée de pixel $x_o + \Delta x$, et $y_o + \Delta y$, est ensuite calculée, comme représenté par le bloc 1145. L'adresse décalée de pixel est ensuite transmise au système de filtrage (bloc 1150) et une détermination est faite pour définir si le pixel dont l'adresse venait juste d'être traitée était ou non le dernier pixel visible d'une trame (losange 1155). Dans la négative, le pixel suivant est attendu et ensuite on entre à nouveau dans le bloc 1135. Lorsque le dernier pixel a été traité, une détermination est faite (losange 1160) pour définir si la dernière trame de la séquence a été ou non traitée. Dans la négative, la trame suivante est attendue et on pénètre à nouveau dans le bloc 1130. Lorsque la dernière trame a été traitée, le programme est terminé.

Sur la figure 12, on a représenté un schéma-bloc d'un système de filtrage temporel 300. Le système de filtrage temporel comprend un premier sous-système de filtrage récurrent 200 et un second sous-système de filtrage récurrent 300. Le sous-système 200 comprend un circuit d'addition 210 qui reçoit, à une entrée, le signal qui est produit par le convertisseur analogique-numérique 151 et qui est désigné par x(t) et il reçoit à son autre entrée un signal désigné par y (t-T), à décrire dans la suite, où T désigne la période de trames vidéo. L'entrée du circuit d'addition 210 qui reçoit le signal x (t) est pondérée par un facteur de pondération $k_1$, et l'entrée du circuit d'addition 210 qui reçoit le signal y (t-T) est pondérée par un facteur de pondération (1-$k_1$). Le signal de sortie du circuit d'addition 210 est un signal désigné par y (t), et ce signal est appliqué à une mémoire numérique de trames 220. La mémoire numérique de trames peut comprendre par exemple une mémoire numérique de trames vidéo modèle FS-963155 fabriquée par THOMSON-CSF Broadcast, Inc. ou bien, en variante, elle peut être constituée par toute mémoire appropriée, comme une mémoire à accès aléatoire, comportant des adresses de pixels (si elles ne sont pas décalées) qui sont fournies par la partie de génération d'adresses du bloc 151 avec génération de signaux de synchronisation et d'horloge. Lorsque des adresses décalées sont utilisées dans des plans d'observations autres que le plan principal, elles sont introduites à partir du processeur 200, comme décrit ci-dessus. Le signal de sortie de la mémoire numérique de trames 200 est constitué dans ce mode de réalisation par le signal désigné par y (t), c'est-à-dire le signal de sortie du circuit d'addition 210.

Le second sous-système de filtrage récurrent 300 comprend un circuit d'addition 310 qui reçoit à une entrée le signal qui est fourni par le signal analogique-numérique 151 et qui est désigné par x (t), et qui reçoit à son autre entrée un signal désigné par z (t-T), à décrire dans la suite. L'entrée du circuit d'addition 310 qui reçoit le signal x (t) est pondérée par un facteur de pondération $k_2$ et l'entrée du circuit d'addition 310 qui reçoit le signal z (t-T) est pondéré par un facteur de pondération (1-$k_2$). Le signal de sortie du circuit d'addition 310 est un signal désigné par z (t) et ce signal est appliqué à une mémoire numérique de trames 320 qui peut être du même type que la mémoire numérique de trames 220 et qui fonctionne d'une manière semblable.

Le signal de sortie y (t) du sous-système 200 et le signal de sortie z (t) du sous-système 300 sont appliqués à un circuit de différence 410 où on obtient la différence y (t) — z (t). Ce signal de différence est appliqué à un convertisseur numérique analogique 195 (figure 1) pour un affichage et/ou un enregistrement.

On peut choisir $k_1$ et $k_2$ de façon à établir une caractéristique temporelle désirée. Par exemple, pour $k_1 = 0,06$ et $k_2 = 0,006$, le système composite répond à des crêtes voisines de 0,1 Hz, où on pourrait s'attendre à obtenir une grande partie de l'information temporelle associée à un « bolus » de contraste passant dans une artère périphérique. La réponse en dessous de 0,003 Hz est essentiellement nulle et elle est très petite au-dessus de 1 Hz.

La présente invention peut être utilisée en coopération avec des techniques qui traitent en outre la série de trames engendrées comme décrit ci-dessus ; par exemple par le type de traitement qui enregistre un suivi de l'opacification maximale de chaque pixel (comme décrit dans la demande de brevet EP-A-0 082 771) et/ou qui conserve un suivi du temps nécessaire jusqu'à opacification maximale (comme décrit dans la demande de brevet EP-A-0 113 605).

L'invention a été décrite en référence à un mode préféré de réalisation mais des variantes sembleront évidentes pour des spécialistes en la matière. Par exemple, le système de filtrage récurrent 300 peut être agencé en cascade en utilisant deux des filtres décrits en série ou en faisant passer les signaux deux fois dans le même système de filtrage. Cela sert, entre autres, à établir une caractéristique globale de filtrage qui est bien moins sensible à la composante de mouvement tomographique.

## Revendications

1. Procédé pour engendrer une image traitée d'une coupe d'un corps, comprenant les étapes consistant à :
- disposer le corps entre une combinaison d'une source de rayonnement et d'un détecteur associé, de manière qu'un faisceau de rayonnement émis par la source arrive angulairement sur ledit corps et passe au travers vers ledit détecteur ;
- produire un mouvement relatif de rotation entre ledit faisceau et ledit corps de manière qu'un plan principal intéressant dudit corps reste sensiblement au foyer pendant ledit mouvement relatif de rotation, caractérisé en ce qu'il consiste à :
- obtenir à partir du détecteur, dans différentes positions angulaires, une série de trames de signaux vidéo électriques qui représentent des images des caractéristiques de transmission de rayonnement du corps à une série d'instants successifs ;
- filtrer temporellement ladite série de trames ; et
- afficher la série de trames filtrées temporellement.

2. Procédé selon la revendication 1, caractérisé en ce qu'il comporte en outre l'étape consistant à injecter un « bolus » d'un agent de contraste dans le corps avant l'obtention des trames de signaux vidéo électroniques.

3. Procédé selon la revendication 1 ou 2, caractérisé en ce que ladite étape de filtrage temporel consiste à appliquer ladite série de trames à un système de filtrage récurrent.

4. Procédé selon la revendication 2 ou 3, caractérisé en ce que ladite étape de filtrage temporel consiste à filtrer la série de trames de signaux vidéo avec une fonction de filtrage ayant une réponse de fréquence temporelle qui correspond sensiblement à la fréquence temporelle du mouvement du « bolus » dans la région en train d'être observée.

5. Procédé selon la revendication 4, caractérisé en ce que le système de filtrage récurrent comprend une paire de filtres récurrents ayant des caractéristiques temporelles différentes.

6. Procédé pour engendrer une image traitée d'une coupe d'un corps, comprenant les étapes consistant à :
- injecter un « bolus » d'un agent de contraste dans le corps ;
- disposer ledit corps entre une combinaison d'une source de rayonnement et d'un détecteur associé, de manière qu'un faisceau de rayonnement émis par la source arrive angulairement sur ledit corps et passe au travers vers ledit détecteur ;
- produire un mouvement relatif de rotation entre ledit faisceau et ledit corps de manière qu'un plan principal intéressant dudit corps reste sensiblement au foyer pendant ledit mouvement relatif de rotation, caractérisé en ce qu'il consiste à :
- obtenir à partir du détecteur, dans différentes positions angulaires, une série de trames de signaux vidéo électriques qui représentent des images des caractéristiques de transmission de rayonnement du corps à une série d'instants successifs, chaque trame comprenant un groupe de pixels, le niveau de signal vidéo pour chaque pixel étant déterminé par la transmissivité de rayonnement d'une région élémentaire du plan principal et de plans adjacents ;
- apporter des transformations géométriques à différentes positions de pixel de niveaux de signal vidéo de trames de la séquence, la transformation géométrique d'un pixel étant une fonction d'un angle relatif de rotation associé à sa trame, et de la distance entre un plan intéressant à observer et le plan principal ;
- filtrer temporellement la série de trames transformées géométriquement ; et
- afficher la série, filtrée temporellement, de trames transformées géométriquement.

7. Procédé selon la revendication 6, caractérisé en ce que l'étape de filtrage temporel consiste à appliquer ladite série de trames à un système de filtrage récurrent.

8. Procédé selon la revendication 7, caractérisé en ce que ledit système de filtrage récurrent comprend une paire de filtres récurrents ayant des caractéristiques temporelles différentes.

9. Procédé selon la revendication 8, caractérisé en ce que ladite étape de filtrage temporel consiste à filtrer la série de trames de signaux vidéo avec une fonction de filtrage ayant une réponse de fréquence temporelle qui correspond sensiblement à la fréquence temporelle du mouvement du « bolus » dans une région à observer.

10. Procédé selon la revendication 7, caractérisé en ce que ladite étape consistant à apporter les transformations géométriques à différentes positions de pixel de niveaux de signal vidéo de trames de la séquence consiste à affecter des adresses modifiées de pixels auxdits niveaux de signal vidéo et à appliquer lesdites adresses modifiées de pixels au système de filtrage récurrent en relation avec lesdits niveaux de signal vidéo.

11. Appareil pour engendrer une image traitée d'une coupe au travers d'un corps, comprenant :
- une combinaison d'une source de rayonnement (110) et d'un détecteur (120) associé accouplés mécaniquement ensemble et positionnés de manière qu'un faisceau de rayonnement émis par la source arrive angulairement sur ledit corps et passe au travers vers le détecteur ;
- un moyen (16) pour produire un mouvement relatif de rotation entre ledit faisceau et ledit corps de manière qu'un plan principal intéressant dudit corps reste sensiblement au foyer pendant ledit mouvement relatif de rotation, caractérisé en ce qu'il comporte ;
- un moyen (130) pour produire à partir du signal de sortie du détecteur, dans différentes positions de rotation, une série de trames de signaux vidéo électroniques qui représentent des images des caractéristiques de transmission de rayonnement du corps à une série d'instants successifs ;

- un moyen (300) pour filtrer temporellement ladite série de trames ; et

- un moyen (198, 199) pour afficher et/ou enregistrer la série, filtrée temporellement, de trames.

12. Appareil selon la revendication 11, caractérisé en ce que ledit moyen de filtrage temporel comprend un système de filtrage récurrent.

13. Appareil selon la revendication 11, caractérisé en ce que ledit moyen de filtrage temporel agit de façon à filtrer la série de trames de signaux vidéo avec une fonction de filtrage ayant une réponse de fréquence temporelle qui correspond sensiblement à la fréquence temporelle du mouvement d'un « bolus » d'une matière de contraste dans la région en train d'être observée.

14. Appareil pour produire une image traitée d'une ou plusieurs coupes d'un corps dans lequel un « bolus » de matière de contraste a été injecté, comprenant :

- une combinaison d'une source de rayonnement (110) et d'un détecteur (120) associé accouplés mécaniquement ensemble et positionnés de manière qu'un faisceau de rayonnement émis par la source arrive angulairement sur ledit corps et passe au travers vers le détecteur ;

- un moyen (16) pour produire un mouvement relatif de rotation entre ledit faisceau et ledit corps de manière qu'un plan principal intéressant dudit corps reste sensiblement au foyer pendant ledit mouvement relatif de rotation, caractérisé en ce qu'il comporte :

- un moyen (130) pour produire à partir du signal de sortie du détecteur, dans différentes positions de rotation, une série de trames de signaux vidéo électroniques qui représentent des images des caractéristiques de transmission de rayonnement du corps à une série d'instants successifs, chaque trame comprenant un groupe de pixels, le niveau de signal vidéo pour chaque pixel étant déterminé par la transmissivité de rayonnement d'une région élémentaire du plan principal et de plans adjacents ;

- un moyen (200) pour apporter une transformation géométrique à différentes positions de pixel de niveaux de signal vidéo de trames de la séquence, la transformation géométrique d'un pixel étant une fonction de l'angle relatif de rotation associé à sa trame et de la distance entre un plan intéressant à observer et le plan principal ;

- un moyen (300) pour filtrer temporellement la série de trames transformées géométriquement ; et

- un moyen (198, 199) pour afficher et/ou enregistrer la série, filtrée temporellement, de trames transformées géométriquement.

15. Appareil selon la revendication 15, dans lequel ledit moyen de filtrage temporel comprend un système de filtrage récurrent.

16. Appareil selon la revendication 15, dans lequel ledit moyen de filtrage temporel agit de façon à filtrer la série de trames de signaux vidéo avec une fonction de filtrage ayant une réponse de fréquence temporelle qui correspond sensiblement à la fréquence temporelle du mouvement d'un « bolus » de matière de contraste dans la région en train d'être observée.

**Claims**

1. A method of producing a processed image of a section taken through a body, comprising the following steps :

- disposing the body between a combination consisting of a source of radiation and of an associated detector in such a manner that a beam of radiation emitted by the source impinge at an angle on the said body and pass therethrough towards the said detector ;

- producing a relative rotational motion between the said beam and the said body in such a manner that a principal plane of interest of the said body remain substantially at the focus during the said rotational relative motion, characterized in that the method consists of :

- obtaining from the detector, at different angular positions, a series of video electric signal frames, which represent images of transmission characteristics of the radiation of the said body at a series of successive instants ;

- time filtering the said series of frames ; and
- displaying the series of time filtered frames.

2. The method as claimed in claim 1, characterized in that it furthermore comprises the step consisting of injecting a bolus of a contrast agent into the body before obtaining electronic video signal frames.

3. The method as claimed in claim 1 or claim 2, characterized in that the said time filtering step consists of applying the said series of frames to a recurrent filtering system.

4. The method as claimed in claim 2 or claim 3, characterized in that the said time filtering step consists of filtering the series of video signal frames with a filtering function having a time frequency response which substantially corresponds to the time frequency of the motion of the bolus in the region in the process of being observed.

5. The method as claimed in claim 4, characterized in that the recurrent filtering system comprises a pair of recurrent filters with different time characteristics.

6. A method for producing a processed image of a section taken through a body comprising the following steps :

- injecting a bolus of a contrast agent into the said body ;

- disposing the said body between the combination of a source of radiation and an associated detector, in such a manner that a beam of the radiation emitted by the source impinge at an angle on the said body and pass therethrough towards the said detector ;

- producing a relative motion between the said beam and the said body in such a manner that a principal plane of interest of the said body remain substantially at the focus during the said relative rotational motion, characterized in that the method consists of :

- obtaining from the detector, in different angular positions, a series of electric video signal frames, which represent transmission characteristics of the radiation of the body at a series of successive instants, each frame comprising a group of pixels, the level of the video signal for each pixel being determined by the transmissivity of the radiation for an elementary region of the principal plane and of the adjacent planes ;

- producing geometrical transformations at different pixel positions for the video signal levels of the frames of the sequence, the geometrical transformation of a pixel being a function of a relative rotational angle associated with its frame, and of the distance between a plane of interest to be observed and the principal plane ;

- time filtering the series transformed frames geometrically ; and

- displaying the series, after time filtering, of the geometrically transformed frames.

7. The method as claimed in claim 6, characterized in that the step of time filtering consists of applying the said series of frames to a recurrent filtering system.

8. The method as claimed in claim 7, characterized in that the said recurrent filtering system comprises a pair of recurrent filters having different time characteristics.

9. The method as claimed in claim 8, characterized in that the said time filtering step consists in filtering the series of video signal frames with a filtering function having a time frequency response which corresponds substantially to the time frequency of the motion of the bolus in a region to be observed.

10. The method as claimed in claim 7, characterized in that the said step consisting of applying the geometrical transformations at different pixel positions of the video signal levels of the frames of the sequence consists of endowing the said levels of the video signal with modified pixel addresses and applying the said modified pixel addresses to the recurrent filtering system in relation with the said levels of the video signals.

11. An apparatus for producing a processed image of a section taken through a body, comprising :

- the combination of a radiation source (110) and of an associated detector (120) coupled mechanically together and positioned in such a manner that a beam of radiation emitted by the source impinge at an angle on the said body and pass therethrough towards the detector ;

- a means (16) to produce a rotational relative motion between the said beam and the said body in such a manner that a principal plane of interest of the said body remain substantially at the focus during the said rotational motion, characterized in that it comprises :

- a means (130) to produce, from the output signal of the detector and in different rotational positions, a series of electronic video signal frames, which represent images of the radiation transmission characteristics of the body at a series of successive instants ;

- a means (300) to time filter the said series of frames ; and

- a means (198 and 199) in order to display and/or record the time filtered series of frames.

12. The apparatus as claimed in claim 11, characterized in that the said time filtering means comprises a recurrent filtering system.

13. The apparatus as claimed in claim 11, characterized in that the said time filtering means acts in such a manner as to filter the series of video signal frames with a filtering function having a time frequency response which substantially corresponds to the time frequency of the motion of a bolus of a contrast material in the region in the process of being observed.

14. An apparatus for producing a processed image of one or more sections of a body, into which a bolus of a contrast material has been injected, comprising :

- the combination of a radiation source (110) and an associated detector (120) which are mechanically coupled together and positioned in such a manner that a beam of radiation emitted by the source impinge at an angle on the said body and pass therethrough towards the detector ;

- a means (16) to produce a rotational relative motion between the said beam and the said body in such a manner that a principal plane of interest of the said body remain substantially at the focus during the said rotational relative motion, characterized in that it comprises :

- a means (130) to produce, starting from the output signal of the detector and in different positions of rotation, a series of electronic video signal frames which represent images of characteristics of transmission of the radiation in the body at a series of successive instants, each frame comprising a group of pixels, the video signal level for each pixel being determined by the radiation transmissivity of an elementary region of the principal plane and of the adjacent ones ;

- a means (200) to produce a geometrical transformation with different pixel positions of the video signal levels of the frames of the sequence, the geometrical transformation of a pixel being a function of the relative rotational angle associated with the frame thereof and of the distance between a plane of interest to be observed and the principal plane ;

- a means (300) to time filter the series of geometrically transformed frames ; and

- a means (198 and 199) to display and/or record the time filtered series of geometrically transformed frames.

15. The apparatus as claimed in claim 15 wherein the said time filtering means comprises a recurrent filtering system.

16. The apparatus as claimed in claim 15, wherein the said time filtering means act in such a manner as to filter the series of video signal frames with a filtering function having a time frequency response which substantially corresponds to the time frequency of the motion of a bolus of contrast material in the region in the process of being observed. ·

**Patentansprüche**

1. Verfahren zum Erzeugen eines verarbeiteten Bildes eines Schnittes durch einen Körper, welches die folgenden Schritte umfaßt, die darin bestehen :

- den Körper zwischen einer Kombination aus einer Strahlungsquelle und einem zugeordneten Detektor anzuordnen, so daß ein von der Quelle ausgestrahltes Strahlenbündel unter einem Winkel auf den genannten Körper auftrifft und auf dem Weg zu dem genannten Detektor ihn durchquert ;

- eine relative Drehbewegung zwischen dem genannten Bündel und dem genannten Körper zu erzeugen, so daß eine interessierende Hauptebene des genannten Körpers im wesentlichen während der genannten relativen Drehbewegung im Brennpunkt bleibt, dadurch gekennzeichnet, daß es darin besteht :

- aus verschiedenen Winkelstellungen eine Reihe von Bildrastern elektrischer Videosignale aus dem Detektor zu gewinnen, welche Bilder der Transmissionsmerkmale des Körpers für die Strahlung zu einer Reihe von aufeinanderfolgenden Zeitpunkten darstellen ;

- die genannte Reihe von Bildrastern zeitlich zu filtern ; und

- die Reihe von zeitlich gefilterten Bildrastern anzuzeigen.

2. Verfahren nach Anspruch 1, dadurch gekennzeichnet, daß es ferner den Schritt umfaßt, der darin besteht, einen « Bolus » eines Kontrastmittels in den Körper zu injizieren, bevor Bildraster der elektronischen Videosignale gewonnen werden.

3. Verfahren nach Anspruch 1 oder 2, dadurch gekennzeichnet, daß der genannte Schritt der zeitlichen Filterung darin besteht, die genannte Reihe von Bildrastern an ein rekurrentes Filtersystem anzulegen.

4. Verfahren nach Anspruch 2 oder 3, dadurch gekennzeichnet, daß der genannte Schritt zur zeitlichen Filterung darin besteht, die Reihe von Bildrastern der Videosignale mit einer Filterfunktion zu filtern, welche eine zeitliche Frequenzantwort aufweist, die der zeitlichen Frequenz der « Bolus »-Bewegung in dem gerade beobachteten Bereich im wesentlichen entspricht.

5. Verfahren nach Anspruch 4, dadurch gekennzeichnet, daß das rekurrente Filtersystem ein Paar rekurrenter Filter umfaßt, welche verschiedene zeitliche Merkmale aufweisen.

6. Verfahren zur Erzeugung eines verarbeiteten Bildes eines Schnittes durch einen Körper, das die Schritte umfaßt, welche darin bestehen :

- einen « Bolus » eines Kontrastmittels in den Körper zu injizieren ;

- den genannten Körper zwischen einer Kombination aus einer Strahlungsquelle und einem zugeordneten Detektor anzuordnen, so daß ein von der Quelle ausgestrahltes Strahlungsbündel unter einem Winkel auf den genannten Körper auftrifft und auf seinem Weg zu dem genannten Detektor ihn durchquert ;

- zwischen dem genannten Bündel und dem genannten Körper eine relative Drehbewegung zu erzeugen, so daß eine interessierende Hauptebene des genannten Körpers während der genannten relativen Drehbewegung im wesentlichen im Brennpunkt bleibt, dadurch gekennzeichnet, daß es darin besteht :

- in verschiedenen Winkelstellungen eine Reihe von Bildrastern der elektrischen Videosignale aus dem Detektor zu gewinnen, welche Bilder der Transmissionsmerkmale des Körpers für die Strahlung zu einer Reihe von aufeinanderfolgenden Zeitpunkten darstellen, wobei jedes Bildraster eine Gruppe von Pixeln umfaßt und der Videosignalpegel für jedes Pixel durch die Strahlungsdurchlässigkeit eines Elementarbereichs der Hauptebene bzw. der daran anschließenden Ebenen bestimmt ist ;

- geometrische Transformationen an verschiedenen Pixelpositionen von Videosignalpegeln von Bildrastern der Folge durchzuführen, wobei die geometrische Transformation eines Pixels von einem seinem Bildraster zugeordneten relativen Drehwinkel und dem Abstand zwischen einer interessierend zu beobachtenden Ebene und der Hauptebene abhängt ;

- die Reihe der geometrisch transformierten Bildraster zeitlich zu filtern ; und

- die Reihe der geometrisch transformierten Bildraster, die zeitlich gefiltert wurde, anzuzeigen.

7. Verfahren nach Anspruch 6, dadurch gekennzeichnet, daß der Schritt des zeitlichen Filterns darin besteht, die genannte Reihe von Bildrastern an ein rekurrentes Filtersystem anzulegen.

8. Verfahren nach Anspruch 7, dadurch gekennzeichnet, daß das genannte rekurrente Filtersystem ein Paar rekurrenter Filter umfaßt, welche verschiedene zeitliche Merkmale aufweisen.

9. Verfahren nach Anspruch 8, dadurch gekennzeichnet, daß der genannte Schritt zum zeitlichen Filtern darin besteht, die Reihe von Bildrastern der Videosignale mit einer Filterfunktion zu filtern, die eine zeitliche Frequenzantwort aufweist, die im wesentlichen der zeitlichen Frequenz der « Bolus »-Bewegung in einem zu beobachtenden Bereich entspricht.

10. Verfahren nach Anspruch 7, dadurch gekennzeichnet, daß der genannte Schritt, bei welchem die geometrischen Transformationen an verschiedenen Pixelpositionen von Videosignalpegeln von Bildrastern der Folge durchgeführt werden, darin besteht, daß veränderte Pixeladressen den Videosignalpegeln zugewiesen werden und die veränderten Pixeladressen an das rekurrente Filtersystem unter Bezugnahme auf diese Videosignalpegel angelegt werden.

11. Gerät zur Erzeugung eines verarbeiteten Bildes eines Schnittes durch einen Körper, mit :

- einer Kombination aus einer Strahlungsquelle (110) und einem zugeordneten Detektor (120), die miteinander mechanisch gekoppelt sind und so angeordnet sind, daß ein von der Quelle ausgestrahltes Strahlungsbündel unter einem Winkel auf den genannten Körper auftrifft und durch diesen hindurch zu dem Detektor gelangt ;

- einem Mittel (16) zur Erzeugung einer relativen Drehbewegung zwischen dem genannten Bündel und dem genannten Körper, so daß eine interessierende Hauptebene des genannten Körpers während der genannten relativen Drehbewegung im wesentlichen im Brennpunkt bleibt, dadurch gekennzeichnet, daß es umfaßt :

- einem Mittel (130), um aus dem Ausgangssignal des Detektors in verschiedenen Drehstellungen eine Reihe von Bildrastern elektronischer Videosignale zu erzeugen, welche Bilder der Transmissionsmerkmale des Körpers für die Strahlung zu einer Reihe von aufeinanderfolgenden Zeitpunkten darstellen ;

- einem Mittel (300) zum zeitlichen Filtern der genannten Reihe von Bildrastern ; sowie

- einem Mittel (198, 199) zum Anzeigen und/oder Speichern der Reihe von Bildrastern, die zeitlich gefiltert worden ist.

12. Gerät nach Anspruch 11, dadurch gekennzeichnet, daß das genannte zeitliche Filtermittel ein rekurrentes Filtersystem umfaßt.

13. Gerät nach Anspruch 11, dadurch gekennzeichnet, daß das genannte zeitliche Filtermittel so arbeitet, daß es die Reihe von Bildrastern der Videosignale mit einer Filterfunktion filtert, welche eine zeitliche Frequenzantwort aufweist, die der zeitlichen Frequenz der Bewegung eines « Bolus » aus einem Kontrastmittel in einem gerade beobachteten Bereich im wesentlichen entspricht.

14. Gerät zur Erzeugung eines verarbeiteten Bildes eines oder mehrerer Schnitte eines Körpers, in welchen ein « Bolus » aus einem Kontrastmittel injiziert worden ist, mit :

- einer Kombination aus einer Strahlungsquelle (110) und einem zugeordneten Detektor (120), welche miteinander mechanisch gekoppelt sind und derart angeordnet sind, daß ein von der Quelle ausgestrahltes Strahlungsbündel unter einem Winkel auf den genannten Körper auftrifft und diesen durchquert und zu dem Detektor gelangt ;

- einem Mittel (16) zum Erzeugen einer relativen Drehbewegung zwischen dem genannten Bündel und dem genannten Körper, so daß eine interessierende Hauptebene des genannten Körpers während der genannten relativen Drehbewegung im wesentlichen im Brennpunkt bleibt, dadurch gekennzeichnet, daß es umfaßt :

- ein Mittel (130), um in verschiedenen Drehstellungen aus dem Ausgangssignal des Detektors eine Reihe von Bildrastern elektronischer Videosignale zu erzeugen, welche Bilder der Transmissionsmerkmale des Körpers für die Strahlung zu einer Reihe von aufeinanderfolgenden Zeitpunkten darstellen, wobei jedes Bildraster eine Gruppe von Pixeln umfaßt und der Videosignalpegel für jedes Pixel durch die Strahlungsdurchlässigkeit eines Elementarbereiches der Hauptebene und der daran anschließenden Ebenen bestimmt wird ;

- ein Mittel (200), um eine geometrische Transformation an verschiedenen Pixelpositionen von Videosignalpegeln von Bildrastern der Folge durchzuführen, wobei die geometrische Transformation eines Pixels eine Funktion des seinem Bildraster zugeordneten relativen Drehwinkels und des Abstands zwischen einer interessierenden zu beobachtenden Ebene und der Hauptebene ist,

- ein Mittel (300) zum zeitlichen Filtern der Reihe von geometrisch transformierten Bildrastern ; und

- ein Mittel (198, 199), um die zeitlich gefilterte Reihe der geometrisch transformierten Bildraster anzuzeigen und/oder zu speichern.

15. Gerät nach Anspruch 14, in welchem das genannte Mittel zum zeitlichen Filtern ein System zum rekurrenten Filtern umfaßt.

16. Gerät nach Anspruch 15, in welchem das genannte Mittel zum zeitlichen Filtern so wirkt, daß die Reihe von Bildrastern der Videosignale mit einer eine zeitliche Frequenzantwort aufweisenden Filterfunktion gefiltert wird, welche der zeitlichen Frequenz der « Bolus »-Bewegung eines Kontrastmittels in dem gerade beobachteten Bereich im wesentlichen entspricht.

# FIG_1

COMMANDE DE TOMOGRAPHIE — 16

SOURCE — 110

20

DETECTEUR — 120

15

130 — GENERATEUR VIDEO

Selection de plan

150 — ENREGISTREUR VIDEO

151 — A/N

adresse

200 — PROCESSEUR

niveau

300 — FILTRE RECURSIF

adresse décalée

198 — VISUALI-SATION

195 — N/A

199 — ENREGIS-TREMENT

# FIG_2

B

A

A

B

A

B

Amplitude

# FIG_3

focal

t ⟶

Amplitude

# FIG_4

non focal

t ⟶

Amplitude

# FIG_5

focal

H$_z$ ⟶

Amplitude

# FIG_6

non focal

1    2    3    4    5

H$_z$ ⟶

FIG_7

Amplitude

Vaisseau dans le plan focal

t →

FIG_8

Amplitude

Vaisseau hors du plan focal

t →

FIG_9

Amplitude

Vaisseau dans le plan focal

0,2      t →

FIG_10

Amplitude

Vaisseau hors du plan focal

1   2   3   t →

3

# FIG_11

VITESSE ANGULAIRE — 1110

POSITION DU PLAN EXAMINE — 1115

CALCUL DE LA DIFFERENCE ANGULAIRE ENTRE TRAMES — 1120

CALCUL DE R — 1125

trame suivante → CALCUL DE $\theta$ — 1130

LECTURE $x_0, y_0$ — 1135 adresse pixel suivant

CALCUL
$\Delta x = R \cos\theta$
$\Delta y = R \sin\theta$ — 1140

CALCUL
$x_0 + \Delta x, y_0 + \Delta y$ — 1145

SORTIE ADRESSE DU PIXEL DECALE VERS SYSTEME DE FILTRAGE — 1150

TEST DERNIER PIXEL — 1155
NON → attendre adresse du pixel suivant
OUI

TEST DERNIERE TRAME — 1160
NON → attendre trame suivante
OUI
FIN

4

# FIG_12

200

$$y(t) = k_1 \, x(t) + (1-k_1) y(t-T)$$

1- $k_1$

$x(t)$

$k_1$

+

210

MEMOIRE TRAME

220

$y(t-T)$

adresse pixel décalé

410

$y(t) - z(t)$
vers 195

niveaux numériques

adresse pixel décalé

310

$k_2$

$x(t)$

+

1- $k_2$

320

MEMOIRE TRAME

$z(t-T)$

$$z(t) = k_2 \, x(t) + (1-k_2) z(t)$$

300